Europäisches Patentamt

European Patent Office

Office européen des brevets

(11) Publication number: **0 272 691 B1**

# EUROPEAN PATENT SPECIFICATION

(45) Date of publication of patent specification: **08.07.92**

(51) Int. Cl.⁵: **G01N 33/535**, G01N 33/58, G01N 33/543

(21) Application number: **87119060.9**

(22) Date of filing: **22.12.87**

(54) **Enzyme immunoassay.**

(30) Priority: **24.12.86 JP 306576/86**

(43) Date of publication of application:
**29.06.88 Bulletin 88/26**

(45) Publication of the grant of the patent:
**08.07.92 Bulletin 92/28**

(84) Designated Contracting States:
**CH DE FR GB IT LI NL**

(56) References cited:
**EP-A- 0 125 893**
**EP-A- 0 132 292**
**WO-A-87/06006**

(73) Proprietor: **FUJIREBIO KABUSHIKI KAISHA also trading as FUJIREBIO INC.**
**6-7, Shimoochiai 4-chome Shinjuku-ku**
**Tokyo 161(JP)**

(72) Inventor: **Ashihara, Yoshihiro**
**Fuchu danchi 2-402 1-28-1 Harumi-cho**
**Fuchu-shi Tokyo(JP)**
Inventor: **Kasahara, Yasushi**
**4-24-6, Hijirigaoka**
**Tama-shi Tokyo(JP)**
Inventor: **Nishizono, Isao**
**Higashinakagami-danki 2-215 1-7-2,**
**Tamagawa-cho**
**Akishima-shi Tokyo(JP)**

(74) Representative: **Kraus, Walter, Dr. et al**
**Patentanwälte Kraus, Weisert & Partner**
**Thomas-Wimmer-Ring 15**
**W-8000 München 22(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

# EP 0 272 691 B1

## Description

This invention relates to an improvement in enzyme immunoassay, and specifically to an improved enzyme immunoassay which can avoid the need for a complex operation of removing an enzyme-labelled antibody or antigen remaining unreacted in a liquid phase out of the assay system by solid-liquid separation and washing before reaction with an enzyme substrate.

Enzyme immunoassay, as is the case with radio-immunoassay (RIA), is a highly sensitive assay method utilizing an antigen-antibody reaction and has been widely used in everyday clinical tests for the detection of drugs or traces of biological components attributed to various diseases which are contained in body fluids such as serum and urine.

The principle of the enzyme immunoassay consists in bonding an enzyme-labelled antibody or antigen to a solid phase by utilizing a non-competitive or competitive reaction of a ligand (or antigen) in a test sample. The enzyme-labelled antibody or antigen reacted with an antibody or antigen fixed to the solid phase is separated from the unreacted enzyme-labelled antibody or antigen remaining in the liquid phase after the antigen-antibody reaction by solid-liquid separation and washing. The degree of the activity of the labelling enzyme bound to the solid phase is detected by reaction with an enzyme substrate.

The enzyme immunoassay itself has been well known in the art, and is described in text books such as Immunoassays in the Clinical Laboratories Techniques (Alan Liss, N. Y. 1979), Method in Enzymology, Vol. 73 (Academic Press, N. Y. 1981) and many papers. Accordingly, in the present specification, these literature references are cited in lieu of a detailed description of the operation of the enzyme immnoassay.

Enzyme immunoassay is roughly classified into non-competitive assay and competitive assay. In view of the complexity of the operation and the need for expert skill, (a) a non-competitive assay using an antibody fixed to a solid phase and a labelled antibody (sandwich assay), and (b) competitive assay using an antigen fixed to a solid phase and a labelled antibody are generally used.

The sandwich assay (a) includes both a one-step and a two-step method. The one-step method involves simultaneously reacting an antibody fixed to a solid phase with a ligand in an assay sample and an enzyme-labelled antibody, removing the unreacted labelled antibody by solid liquid separation and washing after a certain period of time, and measuring the activity of the enzyme bonded to the solid phase. The two-step method, on the other hand, comprises reacting the antibody fixed to the solid phase with the ligand in an assay sample, removing the unreacted ligand in the sample by solid-liquid separation and washing after a certain period of time, then reacting the product with the enzyme-labelled antibody, removing the unreacted enzyme-labelled antibody by solid-liquid separation and washing after a certain period of time, and then measuring the activity of the enzyme bound to the solid phase.

The competitive assay method (b) is a method by which in the step of the antigen-antibody reaction, an enzyme-labelled antigen or antibody and a non-labelled antigen or antibody are reacted competitively with the antigen or antigen fixed to a solid phase, and the amount of the non-labelled antigen is determined.

In any type of the enzyme immunoassays described above, the unreacted enzyme-labelled antibody or antigen remaining in the liquid phase after the end of the antigen-antibody reaction must be removed out of the assay system. In the prior art, the solid phase to which the enzyme labelled antibody or antigen is bound is separated from the liquid phase by solid-liquid separation, and the solid phase is washed to remove the adhering unreacted enzyme-labelled antibody or antigen completely.

However, since this washing operation is very complex and considerably time-consuming, it constitutes an obstacle to adaptation of the enzyme immunoassays to a routine work. Moreover, during the washing operation, bacteria or viruses which may possibly be contained in the assay sample are likely to scatter and might cause danger to the operator. Much research work has therefore been made to avoid the washing operation, but has not resulted in an effective practical method. It has been desired therefore to develop such a method.

The present inventors have made extensive investigations in order to meet such a desire, and have unexpectedly found that when a liquid phase left after the reaction of the labelled antibody or antigen with the antigen of antibody bound to a solid phase is contacted with an insoluble inert carrier having fixed thereto an inhibitor specific to the labelling enzyme, the enzyme in the mobile unreacted enzyme-labelled antibody or antigen remaining in the liquid phase preferentially reacts with the fixed inhibitor and its enzyme activity is reduced. On the other hand, most of the enzyme in the enzyme-labelled antibody or antigen that participates in immunological reaction remains intact and its activity is retained; and that consequently, the complex operation of removing the unreacted enzyme-labelled antibody or antigen by solid-liquid separation and washing can be avoided.

Thus, according to this invention, there is provided a method of enzyme immunoassay for the detection of a ligand in an assay sample which comprises contacting an antibody or antigen fixed to a solid phase

2

and an enzyme-labelled antibody or antigen with the assay sample to induce an antigen-antibody reaction and then reacting the product with an enzyme substrate, wherein the liquid phase after the antigen-antibody reaction is contacted with an insoluble solid carrier having fixed thereto an inhibitor of the labelling enzyme thereby to reduce the activity of the enzyme in the unreacted enzyme-labelled antibody or antigen remaining in the liquid phase.

The inhibitor used in this invention is a substance having the ability to reduce the activity of an enzyme used for labelling an antibody or antigen, and may be selected, according to an enzyme used for labelling, from a variety of substances having the ability to reduce the activity of the enzyme. Generally, non-competitive inhibitors are suitable. Usually, inhibitors having an inhibition constant Ki of the order of at least $10^{-3}$, preferably at least $10^{-5}$, are preferred.

Such inhibitors include, for example, antibodies to enzymes to inhibit the activity (e.g., anti-enzyme antibodies), chelating agents, inhibitors derived from bacteria, and biotin enzyme inhibitors. More specifically, the anti-enzyme antibodies may be for example antibodies to the following enzymes used to label antibodies or antigens in enzyme immunoassay.

Glucohydrases such as amylase, beta-galactosidase, beta-amylase, dextranase and cellulase.

Esterases such as alkaline phosphatase, gamma-glycerophosphatase, choline esterase and lipase.

Peptidases such as trypsin, chymotrypsin, papain, pepsin, plasmin and thrombin.

Dehydrogenases such as glucose-6-phosphate dehydrogenase (G6PDH), alcohol dehydrogenase, maleate dehydrogenase and lactate dehydrogenase.

Biotin enzymes such as carboxylase, transcarboxylase, pyruvate carboxylase and propionyl CoA carboxylase.

Oxidases such as peroxidase, catalase and glutathion peroxidase.

The chelating agents may be, for example, EDTA, EGTA, phenanthroline, and derivatives of these. Inhibitors derived from bacteria and biotin enzyme inhibitors can also be used.

Specific examples of a combination of such an inhibitor and an enzyme are listed below.

| Labelling enzyme | Enzyme inhibitor |
|---|---|
| Biotin enzyme | Avidin or streptoavidin |
| Maleate dehydrogenase | Bacteria-derived inhibitor |
| Amylase | Bacteria-derived inhibitor |
| Amylase | Anti-amylase antibody |
| Amylase | EDTA |
| Alkaline phosphatase | EDTA |
| Gamma-glycerophosphatase | Anti-glycerophophatase antibody |
| Peroxidase | Hydrogen peroxide |
| Trypsin | Trypsin inhibitor |
| Dextranase | Anti-dextranase antibody |
| Alpha-amylase | Wheat-derived anmylase inhibitor or bacteria-derived inhibitor |
| Trypsin | Soybean-derived trypsin inhibitor |
| Propionyl CoA carboxylase | Avidin or streptoavidin |
| Viotin enzyme | Avidin or streptoavidin |
| Thrombin | Alpha-antitrypsin |
| Plasmin or chymotrypsin | Plasmin inhibitor |

In accordance with this invention, the enzyme inhibitor is used in a form fixed to an insoluble solid carrier. The carrier may be any of a wide variety of solid materials which are substantially insoluble in a liquid phase in an antigen-antibody reaction used in enzyme immunoassay. Generally, the solid materials include, for example, polymeric porous gel particles, polymer latices, polymeric porous membranes, and fibrous sheets. More specifically, examples of the polymeric porous gel particles are activated dextran gels such as CNBr Sepharose®, CNBr Sephadex®, epoxy Sepharose®, thiol Sepharose®, thiol Sephadex®, carboxy cellulose, carboxy Sepharose® and carbox Sephadex®, and various ion-exchange resin particles. Examples of the polymer latices are an aminated polystyrene latex and a carboxylated polystyrene latex. Conveniently, these polymeric porous gel particles and polymer latices have an average particle diameter in the range of 0.2 to 100 micrometers, preferably 1 to 10 micrometers.

The polymeric porous membranes and fibrous sheets may be for example "membrane filters", such as a nitrocellulose filter, a cellulose acetate filter, filter paper, a glass fiber filter, and various ion-exchange membranes. Desirably, they have an average pore size of 0.2 to 3 micrometers, preferably 1 to 3 micrometers.

Fixation of the enzyme inhibitor to the carrier may be effected by physical adsorption, it is generally preferable, however, to bond covalently a functional group present on, or introduced into, the surface of the carrier (for example, an amino group, a carboxyl group or a hydroxyl group) to a functional group present in the enzyme inhibitor (for example, an amino group, a carboxyl group, a hydroxyl group, a thiol group or an azide group) using carbodiimide either directly or through a bifunctional crosslinking agent such as a dialdehyde, a quinone, difluorobenzene or a diisocyanate). Such a fixation method is well known in the art, and can be successfully carried out by known procedures described, for example, in J. Biochem., 82, 261-266 (1987), Tokyo, and Eur. J. Biochem., 71, 459-467 (1978).

The amount of the inhibitor to be fixed to the carrier is not critical, and may be varied with the type of the inhibitor or the type of the enzyme to be inhibited. Any skilled person can easily determine the optimum amount of the inhibitor by conducting a simple routine experiment. Generally, the inhibitor may be added in a large excess with respect to the amount of a labelled antigen or antibody.

The carrier having the enzyme inhibitor fixed thereto is added to, and thus contacted with, the liquid phase left after the antigen-antibody reaction in the enzyme immunoassay. Consequently, the inhibitor on the carrier reacts with the enzyme in the labelled antibody or antigen remaining unreacted in the liquid phase to reduce the activity of the enzyme. This reaction for reducing the enzyme activity is usually carried out at room temperature or 37°. If desired, it may be carried out at an elevated temperature up to about 45°C.

Addition of the carrier having the enzyme inhibitor bound thereto to the liquid phase may be effected prior to, or simultaneously with, the addition of an enzyme substrate.

As a result of addition of the carrier, the mobile unreacted enzyme-labelled antibody or antigen preferentially reacts with the inhibitor on the carrier and, the activity of the enzyme on the labelled antibody or antigen is reduced. During this time, part of the enzyme with antibody or antigen bound to the solid phase may make contact with, temporarily, the inhibitor on the carrier. This temporary bonding is easily dissociated by mechanical forces such as agitation or shaking after the addition of the enzyme substrate, and the inherent activity of the enzyme is restored. Hence, that part of the enzyme, which has been bound temporarily, can participate in the reaction with the substrate for enzyme, and no substantial effect is believed to be exerted in the enzyme immunoassay by the temporary binding. The contacting of the solid phase with the carrier might result in inhibition of part of the enzyme in the labelled antibody or antigen bound to the solid phase. Microscopically, however, only a very small portion of the solid phase makes contact with the carrier, and a greater portion of the labelled antibody or antigen bound to the solid phase remains intact. For this reason, too, such a contacting is believed to exert no deleterious effect in the enzyme immunoassay.

Accordingly, the unreacted enzyme-labelled antibody or antigen can be removed from the reaction system involving the substrate for enzyme without performing the complex solid-liquid separation and washing operation.

Usually, the enzyme substrate can be added to, reacted with, the liquid phase kept in contact with the carrier having the enzyme inhibitor fixed thereto. If desired, a solution of the enzyme substrate may be added to a solid phase left after removal of the liquid phase by solid-liquid separation. The reaction temperature at this time is generally about 4 °C to about 40 °C, preferably room temperature to about 37 °C.

The method of this invention described hereinabove, makes it possible to avoid the complexity of the washing operation which has previously been regarded as an inevitable defect of enzyme immunoassays. By a simple and convenient operation, a ligand in sample can be measured with good sensitivity and within a short period of time by the method of this invention. Examples of ligands that can be detected by the method of this invention include haptens such as drugs, steroid, hormones, vitamins and physologically active amines; cancer-related antigens such as AFP, ferritin, TPA and CEA; plasma proteins such as albumin, IgG, IgM and coagulation factors, viruses such as HBs, HA, HBe, cytomegaro, herpes simplex, EB and papilloma; and bacteria such as Chlamydia gonorrhea.

The following Examples illustrate the method of this invention more specifically.

EXAMPLE 1

1. Preparation of a porcine alpha-amylase from porcine pancrea conjugate with theophylline:

Ten milligrams of 8-propylcarboxytheophylline and 5 mg of N-hydroxysuccinimide were dissolved in 500 microliters of dimethylformamide, and 10 mg of water-soluble carbodiimide (EDC) was added. The mixture was stirred at room temperature for 3 hours. Two hundred microliters of the reaction solution was added to 2 ml of a 0.5% alpha-amylase from porcine pancrea solution (pH 7.5) containing 0.1M glycerophosphoric acid and 1% starch. The mixture was left to stand at room temperature for 2 hours. The reaction solution was applied onto a column (1 cm × 35 cm) of Sephadex® G-25 equilibrated with 20 mM glycerophosphoric acid, pH 7.0 to remove the unreacted substances. Thereby, 7 mg of the desired theophylline-amylase conjugate was obtained. BSA was added to the product to adjust the final concentration to 1 % to stabilize the conjugate.

2. Preparation of a carrier binding an amylase inhibitor:

Epoxy Sepharose 4B® was swollen with water, and washed. Five ml of the epoxy Sepharose® 4B was suspended in 5 ml of an amylase inhibitor solution (0.1M carbonic acid, pH 9.5) containing 10 mg of the inhibitor per ml, and the mixture was stirred at room temperature for one day. The gel was fully washed with 0.02M phosphate-buffered physiological saline (PBS), and then suspended in 0.1M tris-HCl (pH 8.5). The suspension was left to stand at 37 °C for 2 hours. It was washed with PBS four times, and re-suspended in 10 ml of PBS containing 1% BSA to prepare the carrier bound with the amylase inhibitor.

3. Preparation of Sepharose® bound with anti-theophylline antibody:

Fifteen milligrams of anti-theophylline mouse monoclonal antibody was dissolved in 5 ml of a buffer at pH 8.0 containing 0.1M carbonate and 2.5M NaCl. Two grams of CNBr-activated Sepharose® (made by Pharmacia Co.) was swollen with 1mM aqueous HCl solution and washed with the same solution anti-theophylline antibody was added. The mixture was stirred at room temperature for 18 hours by an end-over-end mixer, and then washed five times with PBS. Then, the mixture was suspended in 0.1M tris-HCl (pH 8.5) and left to stand at room temperature for 2 hours. Thereafter, the mixture was washed with PBS. The gel thus obtained was stored as suspended in 15 ml of a 1% BSA-PBS solution.

4. Measurement of theophylline

To 50 microliters (0 to 100 micrograms/ml) of a solution of theophylline were added 50 microliters (20 micrograms/ml) of the theophylline-amylase conjugate prepared in 1. Fifty microliters of the gel prepared in 3, and the mixture was left to stand at 37 °C for 10 minutes. Then, 100 microliters of the amylase inhibitor-fixed carrier prepared in 2 and 800 microliters of a 50mM glycerophosphate buffer at pH 7.0 containing 1% of p-nitrophenyl hexose and 3 units/ml of alpha-glycosidase were added to the resulting gel, and the enzyme reaction was carried out for 30 minutes.

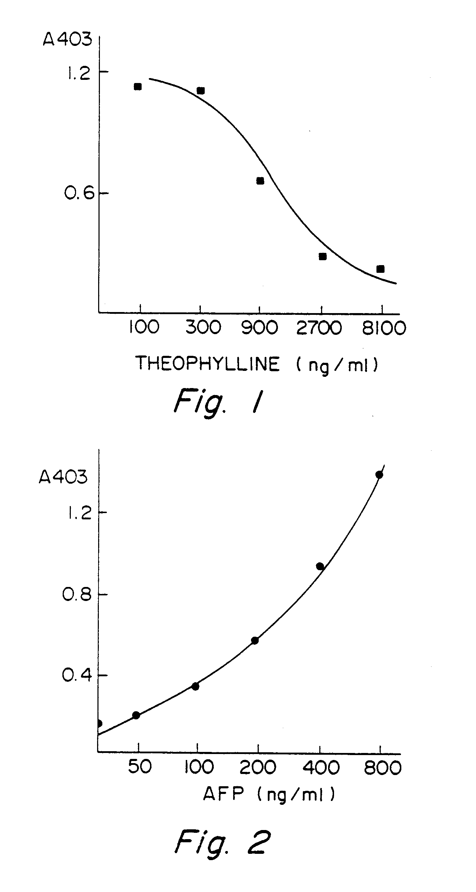

Fig. 1 shows the relation between the activity of the enzyme and the concentration of theophylline in the above reaction.

EXAMPLE 2

1. Preparation of an anti-human alpha-fetoprotein antibody bound filter

An activated membrane filter (immunoaffinity membrane, 3 $\mu$m pore; made by Biodyne Inc.) was cut to a size of 10 cm × 10 cm, and immersed in 20 ml of an anti-human alpha-fetoprotein mouse monoclonal antibody (200 micrograms/ml mouse IgG, PBS solution), left to stand overnight at 4 °C, and washed with PBS. Then, it was immersed in 20 ml of a 0.1M tris-HCl (pH 8.5) containing 5 % BSA to block the active groups completely. The filter thus obtained was punched out to form a disc in 5 mm of diameter.

2. Preparation of an anti-human alpha-fetoprotein mouse IgG-Fab/porcine alpha-amylase conjugate

Two milliliters of 0.1M acetate buffer (pH 4.2) and 300 micrograms of pepsin were added to 10 ml of anti-human alpha-fetoprotein mouse IgG, and the mixture was stirred at 37 °C for 18 hours. 0.1M NaOH was added to adjust the pH of the mixture at 7.0, and the reaction solution was applied onto a colum of Sephacryl® S-300 equilibrated with 0.1M phosphate-buffered 1 mM EDTA solution at pH 6.3. The column was eluted with the above-mentioned buffer. Peak fractions eluted at a molecular weight of about 100,000 daltons were collected and concentrated to 1 ml to obtain anti-human alpha-fetoprotein mouse IgG F(ab')$_2$.

To 1 ml of F(ab')$_2$ thus obtained containing 3 mg protein was added 100 microliters of an aqueous solution (pH 7.0) of 2-mercaptoethylamine (10 mg/ml), and the mixture was stirred at 37 °C for 90 minutes. The reaction solution was gel-filtered on a column of Sephadex® G-25 equilibrated with 0.1M phosphate buffer (pH 7.0) to remove the unreacted 2-mercaptoethylamine and to obtain HS-Fab'. Porcin alpha-amylase(3 mg) was dissolved in 1ml of 0.1M phosphate buffer, 200 microliters of a 3% aqueous solution of starch and 200 ml of a DMF solution of CHMS (1 mg/ml). The mixture was left to stand at room temperature for 1 hour to allow the reaction to proceed. The reaction solution was gel-filtered on a column of Sephadex® G-25 equilibrated with 0.1M phosphate buffer (pH 7.0) to remove the unreacted CHMS and obtain 2.5 mg of CHM-reacted amylase. Two milligrams of the HS-Fab thus obtained was added to 2 mg of the CHM-treated amylase, and the mixture left sto stand at 4 °C for 18 hours. One hundred microliters of an aqueous solution of 2-mercaptoethylamine (10 mg/ml) was added, and the mixture was stirred at 37 °C for 1 hour. The reaction solution was applied onto a column of Sephacryl® S-300 gel (1 cm × 100 cm) equilibrated with 20 mM glycerophosphate-buffered physiological saline (GBS), pH 7.0, and protein components having a molecular weight in the neighborhood of 100,000. Thus, 10 ml of the desired Fab'-S-CHM-amylase solution (300 micrograms/ml) was obtained.

3. Measurement of human alpha-fetoprotein

One 5 cm-diameter anti-human alpha-fetoprotein antibody-fixed filter (prepared in Example 2, 1) was taken into a pilot tube, and 50 microliters of a human alpha-fetoprotein solution (0 to 800 micrograms/ml). Furthermore, 50 microliters (3 micrograms/ml) of the anti-human alpha-fetoprotein mouse IgG Fab-amylase bound product (prepared in Example 2, 2) was added. The mixture was incubated at 37 °C for 30 minutes. Then, 100 microliters of the amylase inhibitor gel prepared in Example 1, 2 and 800 microliters of a substrate solution (pH 7.0) containing 1 % of p-nitrophenylhexose and 3 units/ml of alpha-glycosidase were added, and the mixture was reacted at 37 °C for 30 minutes. The reaction was stopped by adding 500 microliters of 0.1M EDTA solution at pH 9.0 containing 0.5M carbonate. Absorption of the color of the supernatant was measured 403 nm. Figure 2 shows a standard curve drawn on the basis of the results obtained.

## Claims

1. A method of enzyme immunoassay for the detection of a ligand in an assay sample which comprises contacting an antibody or antigen fixed to a solid phase and an enzyme-labelled antibody or antigen with the assay sample to induce an antigen-antibody reaction and then reacting the product with an enzyme substrate, wherein the liquid phase after the antigen-antibody reaction is contacted with an insoluble solid carrier having fixed thereto an inhibitor of the labelling enzyme thereby to reduce the activity of the enzyme in the unreacted enzyme-labelled antibody or antigen remaining in the liquid phase.

2. The method of claim 1 wherein the enzyme immunoassay is sandwich enzyme immunoassay.

3. The method of claim 1 wherein the enzyme immunoassay is competitive assay.

4. The method of claim 1 wherein an antibody fixed to a solid phase and a labelled antibody are used.

5. The method of claim 1 wherein an antibody fixed to a solid phase and a labelled antigen are used.

6. The method of claim 1 wherein the insoluble solid carrier is a polymeric porous particulate gel, a polymer latex, a polymeric porous membrane or a fibrous sheet.

7. The method of claim 6 wherein the insoluble solid carrier is a polymeric porous particulate gel or a polymer latex having an average particle diameter in the range of 0.2 to 100 micrometers.

8. The method of claim 6 wherein the insoluble solid carrier is a polymeric porous membrane or a fibrous sheet having an average pore diameter in the range of 0.2 to 3 micrometers.

9. The method of claim 1 wherein the inhibitor is an anti-enzyme antibody, a chelating agent, a bacteria-derived inhibitor, or a biotin enzyme inhibitor.

10. The method of claim 1 wherein the insoluble solid carrier to which the inhibitor is fixed is contacted with the liquid phase before it is reacted with the enzyme substrate.

11. The method of claim 1 wherein the insoluble solid carrier to which the inhibitor is fixed is contacted with

EP 0 272 691 B1

the liquid phase simultaneously with its reaction with the enzyme substrate.

**Revendications**

1. Méthode de dosage immuno-enzymatique pour la détection d'un ligand dans un échantillon à analyser, qui consiste à mettre en contact un anticorps ou antigène fixé à une phase solide et un anticorps ou antigène marqué par une enzyme avec l'échantillon à analyser pour provoquer une réaction antigène-anticorps, puis à faire réagir le produit avec un substrat d'enzyme, dans laquelle la phase liquide, après la réaction antigène-anticorps, est mise en contact avec un support solide insoluble auquel est fixé un inhibiteur de l'enzyme de marquage pour réduire ainsi l'activité de l'enzyme associée à l'anticorps ou antigène marqué par une enzyme qui reste sans avoir réagi dans la phase liquide.

2. Méthode selon la revendication 1, dans laquelle le dosage immuno-enzymatique est un dosage immuno-enzymatique en sandwich.

3. Méthode selon la revendication 1, dans laquelle le dosage immuno-enzymatique est un dosage par compétition.

4. Méthode selon la revendication 1, dans laquelle on utilise un anticorps fixé à une phase solide et un anticorps marqué.

5. Méthode selon la revendication 1, dans laquelle on utilise un anticorps fixé à une phase solide et un antigène marqué.

6. Méthode selon la revendication 1, dans laquelle le support solide insoluble est un gel polymère poreux en particules, un latex de polymère, une membrane polymère poreuse ou une feuille fibreuse.

7. Méthode selon la revendication 6, dans laquelle le support solide insoluble est un gel polymère poreux en particules ou un latex de polymère, ayant un diamètre moyen de particules compris dans l'intervalle de 0,2 à 100 micromètres.

8. Méthode selon la revendication 6, dans laquelle le support solide insoluble est une membrane polymère poreuse ou une feuille fibreuse, ayant un diamètre moyen de pores compris dans l'intervalle de 0,2 à 3 micromètres.

9. Méthode selon la revendication 1, dans laquelle l'inhibiteur est un anticorps anti-enzyme, un agent chélateur, un inhibiteur d'origine bactérienne ou un inhibiteur d'enzyme à base de biotine.

10. Méthode selon la revendication 1, dans laquelle le support solide insoluble auquel est fixé l'inhibiteur est mis en contact avec la phase liquide avant que celle-ci soit mise à réagir avec le substrat d'enzyme.

11. Méthode selon la revendication 1, dans laquelle le support solide insoluble auquel est fixé l'inhibiteur est mis en contact avec la phase liquide en même temps que celle-ci est mise à réagir avec le substrat d'enzyme.

**Patentansprüche**

1. Enzymimmunoassay-Verfahren zur Detektion eines Liganden in einer Testprobe, dadurch **gekennzeichnet,** daß man einen festphasengebundenen Antikörper oder ein festphasengebundenes Antigen und einen enzymmarkierten Antikörper oder ein enzymmarkiertes Antigen mit der Testprobe in Kontakt bringt, um eine Antigen-Antikörper-Reaktion zu induzieren, und dann das Produkt mit einem Enzymsubstrat umsetzt, wobei die flüssige Phase nach der Antigen-Antikörper-Reaktion mit einem unlöslichen festen Träger in Kontakt gebracht wird, auf den ein Inhibitor des markierenden Enzyms fixiert ist, um dadurch die Aktivität des Enzyms in dem nicht umgesetzten, enzymmarkierten Antikörper oder Antigen, welcher bzw. welches in der flüssigen Phase verbleibt, zu reduzieren.

2. Verfahren nach Anspruch 1, dadurch **gekennzeichnet,** daß der Enzymimmunoassay ein Sandwich-

8

Enzymimmunoassay ist.

3. Verfahren nach Anspruch 1, dadurch **gekennzeichnet,** daß der Enzymimmunoassay ein kompetitiver Assay ist.

4. Verfahren nach Anspruch 1, dadurch **gekennzeichnet,** daß ein festphasengebundener Antikörper und ein markierter Antikörper verwendet werden.

5. Verfahren nach Anspruch 1, dadurch **gekennzeichnet,** daß ein festphasengebundener Antikörper und ein markiertes Antigen verwendet werden.

6. Verfahren nach Anspruch 1, dadurch **gekennzeichnet,** daß der unlösliche feste Träger ein polymeres poröses teilchenförmiges Gel, ein polymerer Latex, eine polymere poröse Membran oder eine faserartige Schicht ist.

7. Verfahren nach Anspruch 6, dadurch **gekennzeichnet,** daß der unlösliche feste Träger ein polymeres poröses teilchenförmiges Gel oder ein polymerer Latex mit einem durchschnittlichen Teilchendurchmesser im Bereich von 0,2 bis 100 Mikrometer ist.

8. Verfahren nach Anspruch 6, dadurch **gekennzeichnet,** daß der unlösliche feste Träger eine polymere poröse Membran oder eine faserartige Schicht mit einem durchschnittlichen Porendurchmesser im Bereich von 0,2 bis 3 Mikrometer ist.

9. Verfahren nach Anspruch 1, dadurch **gekennzeichnet,** daß der Inhibitor ein Anti-Enzymantikörper, ein chelatbildendes Reagenz, ein Inhibitor bakterieller Herkunft oder ein Biotin-Enzyminhibitor ist.

10. Verfahren nach Anspruch 1, dadurch **gekennzeichnet,** daß der unlösliche feste Träger, an welchem der Inhibitor fixiert ist, mit der flüssigen Phase vor Reaktion mit dem Enzymsubstrat in Kontakt gebracht wird.

11. Verfahren nach Anspruch 1, dadurch **gekennzeichnet,** daß der unlösliche feste Träger, an welchem der Inhibitor fixiert ist, mit der flüssigen Phase gleichzeitig mit dessen Reaktion mit dem Enzymsubstrat in Kontakt gebracht wird.

Fig. 1

Fig. 2